(19) Europäisches Patentamt
European Patent Office
Office européen des brevets

(11) Veröffentlichungsnummer: **0 086 411**
**A2**

(12) # EUROPÄISCHE PATENTANMELDUNG

(21) Anmeldenummer: **83101046.7**

(22) Anmeldetag: **04.02.83**

(51) Int. Cl.³: **C 07 D 239/10**, A 01 N 43/54

(30) Priorität: **16.02.82 JP 22109/82**

(43) Veröffentlichungstag der Anmeldung: **24.08.83**
**Patentblatt 83/34**

(84) Benannte Vertragsstaaten: **AT BE CH DE FR GB IT LI NL SE**

(71) Anmelder: **NIHON TOKUSHU NOYAKU SEIZO K.K.,**
**No.4, 2-Chome, Nihonbashi Honcho, Chuo-ku**
**Tokyo 103 (JP)**

(72) Erfinder: **Aya, Masahiro, 2-844, Suzuki-cho, Kodaira-shi Tokyo (JP)**
Erfinder: **Saito, Junichi, 3-7-12, Osawa, Mitaka-shi Tokyo (JP)**
Erfinder: **Yasui, Kazuomi, 7-6-15, Shakujii-dai, Nerima-ku Tokyo (JP)**
Erfinder: **Shiokawa, Kozo, 210-6, Shukugawara Tama-ku, Kawasaki-shi Kanagawa-ken (JP)**

(74) Vertreter: **Schumacher, Günter, Dr. et al, c/o Bayer AG**
**Zentralbereich Patente, Marken und Lizenzen,**
**D-5090 Leverkusen 1 Bayerwerk (DE)**

(54) **Methyl-substituierte Tetrahydro-2-pyrimidinon-Derivate, Verfahren zu ihrer Herstellung und ihre Verwendung als Herbizide sowie neue Zwischenprodukte für ihre Herstellung.**

(57) Methyl-substituierte Tetrahydro-2-pyrimidinon-Derivate der allgemeinen Formel

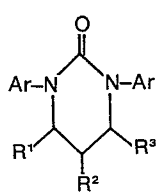

(I)

in der

Ar jeweils unabhängig voneinander für eine Aryl-Gruppe steht, die gegebenenfalls mono- oder polysubstituiert ist durch einen oder mehrere Substituenten, ausgewählt aus Halogen, $C_1$- bis $C_6$-Alkyl, $C_1$- bis $C_6$-Alkoxy, $C_1$- bis $C_6$-Alkylthio, Nitro, Phenoxy und Trifluoromethyl, und

$R^1$, $R^2$ und $R^3$ unabhängig voneinander für ein Wasserstoff-Atom oder eine Methyl-Gruppe stehen, mit der Maßgabe, daß mindestens einer der Substituenten $R^1$, $R^2$ und $R^3$ eine Methyl-Gruppe darstellt, sind neu und finden als Herbizide Verwendung, insbesondere als selektive Herbizide, die gegen Unkräuter in solchen Nutzpflanzen wie Baumwolle und Reis eingesetzt werden können.

Die N,N'-Diaryl-N-halogenoalkylharnstoffe, die Ausgangsstoffe für die Herstellung der Verbindungen der Formel (I) sind, sind ebenfalls neu.

EP 0 086 411 A2

NIHON TOKUSHU NOYAKU SEIZO K.K.

Tokoy, Japan

Bi/Bck
I b

# Methyl-substituierte Tetrahydro-2-pyrimidinon-Derivate, Verfahren zu ihrer Herstellung und ihre Verwendung als Herbizide sowie neue Zwischenprodukte für ihre Herstellung

Die vorliegende Erfindung betrifft bestimmte neue methyl-substituierte Tetrahydro-2-pyrimidinon-Derivate, ein Verfahren zu ihrer Herstellung und ihre Verwendung als Herbizide.

Die vorliegende Erfindung betrifft auch neue Zwischenprodukte für die Herstellung der neuen aktiven Derivat-Verbindungen sowie ein Verfahren zur Herstellung derartiger Zwischenprodukte.

Die Verbindungen 1-Methyl-3-phenylhexahydro-2-pyrimidinon und 1-Methyl-3-phenyl-5-methyl-2-imidazolidinon sind in Chem. Abstracts 57, 9860a (1962) offenbart; dort findet sich jedoch kein Hinweis bezüglich ihrer Verwendbarkeit. Tests ähnlich denjenigen, die in dieser Beschreibung später erläutert werden, zeigen, daß diese Verbindungen kaum eine herbizide Wirksamkeit besitzen.

Die vorliegende Erfindung macht nunmehr als neue Verbindungen die methyl-substituierten Tetrahydro-2-pyrimidinon-Derivate der allgemeinen Formel

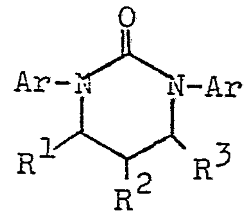

(I)

verfügbar, in der
Ar jeweils unabhängig voneinander für eine Aryl-Gruppe steht, die gegebenenfalls mono- oder polysubstituiert ist durch einen oder mehrere Substituenten ausgewählt aus Halogen, $C_1$- bis $C_6$-Alkyl, $C_1$- bis $C_6$-Alkoxy, $C_1$- bis $C_6$-Alkylthio, Nitro, Phenoxy und Trifluoromethyl, und
$R^1$, $R^2$ und $R^3$ unabhängig voneinander für ein Wasserstoff-Atom oder eine Methyl-Gruppe stehen, mit der Maßgabe, daß mindestens einer der Substituenten $R^1$, $R^2$ und $R^3$ eine Methyl-Gruppe darstellt.

Die vorliegende Erfindung macht weiterhin ein Verfahren zur Herstellung einer Verbindung der vorliegenden Erfindung verfügbar, das dadurch gekennzeichnet ist, daß eine Verbindung der allgemeinen Formel

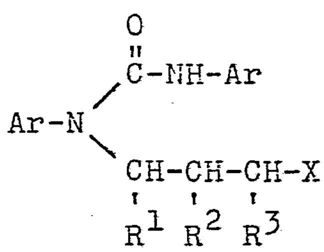

(II)

,

in der
Ar, $R^1$, $R^2$ und $R^3$ die im Vorstehenden angegebenen Bedeutungen haben und
X für ein Halogen-Atom steht,
mit einem Alkalimetallhydroxid der allgemeinen Formel

$$MOH \qquad (III),$$

in der

M für ein Alkalimetall-Atom steht,

zur Umsetzung gebracht wird.

Die neuen methyl-substituierten Tetrahydro-2-pyrimidinon-Derivate der vorliegenden Erfindung zeichnen sich durch eine hervorragende selektive herbizide Wirksamkeit aus.

Die vorliegende Erfindung macht weiter als neue Zwischenprodukte die N,N'-Diaryl-N-halogenoalkyl-harnstoffe der im Vorstehenden angegebenen allgemeinen Formel (II) verfügbar.

Die vorliegende Erfindung macht weiterhin ein Verfahren zur Herstellung einer Zwischenprodukt-Verbindung der Formel (II) gemäß der vorliegenden Erfindung verfügbar, das dadurch gekennzeichnet ist, daß ein Amin der allgemeinen Formel

$$Ar\text{-}NH\text{-}\overset{R^1}{\overset{|}{CH}}\text{-}\overset{R^2}{\overset{|}{CH}}\text{-}\overset{R^3}{\overset{|}{CH}}\text{-}X \qquad (IV),$$

in der Ar, $R^1$, $R^2$, $R^3$ und X die im Vorstehenden angegebenen Bedeutungen haben,

mit einem Isocyanat der allgemeinen Formel

$$Ar\text{-}N{=}C{=}O \qquad (V),$$

in der Ar die im Vorstehenden angegebene Bedutung hat und gleich dem oder verschieden von dem Ar-Rest in dem Amin der Formel (IV) sein kann,

zur Umsetzung gebracht wird.

Wiewohl die vorgenannten, in der Literatur beschriebenen Verbindungen nur eine sehr geringe herbizide Wirksamkeit besitzen, wie die im Nachstehenden mitgeteilten Test-Ergebnisse zeigen, wurde nunmehr gefunden, daß die Verbindungen der Formel (I) gemäß der vorliegenden Erfindung überraschenderweise eine ausgezeichnete selektive herbizide Aktivität aufweisen und eine genaue Bekämpfungswirkung auf unerwünschte Unkräuter bei wertvollen Nutzpflanzen wie Baumwolle und Reis ausüben, und zwar ohne jegliche Phytotoxizität gegenüber den Nutzpflanzen. Angesichts der Tatsache, daß die Verbindungen der Formel (I) gemäß der vorliegenden Erfindung ein vollständiges Weißwerden und Verwelken der Stiele und Blätter der Unkräuter herbeiführen, wird angenommen, daß die Wirksamkeit der Verbindungen hauptsächlich auf einem Mechanismus beruht, der die Chlorophyll-Synthese hemmt. Infolgedessen beeinträchtigen die Verbindungen gemäß der vorliegenden Erfindung, unter Betrachtung dieses Mechanismus, Baumwolle, Reis und andere Nutzpflanzen nicht.

Damit bewirkt die vorliegende Erfindung einen bedeutsamen technischen Fortschritt.

Bevorzugte Verbindungen der Formel (I) gemäß der vorliegenden Erfindung sind solche Verbindungen, in denen Ar für eine Phenyl- oder α-Naphthyl-Gruppe steht, die
gegebenenfalls mono- oder polysubstituiert ist durch einen oder mehrere Substituenten ausgewählt aus Fluor, Chlor, Brom, Iod, Methyl, Ethyl, Propyl, Isopropyl, n-Butyl, Isobutyl, sec-Butyl, tert-Butyl, Methoxy, Ethoxy, Propoxy, Isopropoxy, n-Butoxy, Isobutoxy, sec-Butoxy, tert-Butoxy, Methylthio, Ethylthio, Propylthio, Isopropylthio, n-Butylthio, Isobutylthio, sec-Butylthio, tert-Butylthio, Nitro, Phenoxy und Trifluoromethyl, und

$R^1$, $R^2$ und $R^3$ die im Vorstehenden angegebenen Bedeutungen haben.

Besonders bevorzugte Verbindungen der Formel (I) sind solche Verbindungen, in denen mindestens einer der Reste Ar einen Substituenten in der Meta-Stellung trägt.

Die Reaktion gemäß der vorliegenden Erfindung zur Herstellung der Verbindungen der Formel (I) kann durch die folgende Gleichung beschrieben werden

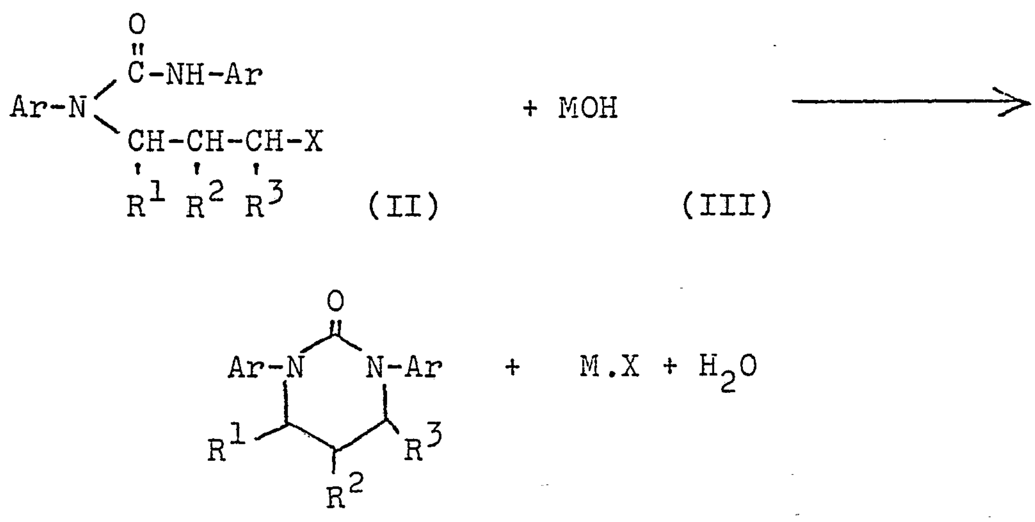


(II) (III)

(I)

(in der Ar, $R^1$, $R^2$, $R^3$, X und M die im Vorstehenden angegebenen Bedeutungen haben).

Bevorzugte Ausgangsmaterialien der Formel (II) sind diejenigen Verbindungen, in denen Ar, $R^1$, $R^2$ und $R^3$ die im Vorstehenden bei der Definition bevorzugter und besonders bevorzugter Verbindungen der Formel (I) angegebenen Bedeutungen haben und X für ein Fluor-, Chlor-, Brom- oder Iod-Atom steht.

Beispiele für die Verbindungen der Formel (II), die zu erwähnen sind, umfassen:
N-/¯3-Chloro- (oder -Bromo-) -2-methylpropyl_7-N-phenyl-N'-fluoro- (oder -chloro- oder -bromo-) -phenylharnstoff,
N-/¯3-Chloro- (oder -Bromo-) -2-methylpropyl_7-N-3-fluoro- (oder -chloro- oder -bromo-) -phenyl-N'-phenylharnstoff,
N-/¯3-Chloro- (oder -Bromo-) -2-methylpropyl_7-N-phenyl-N'-3-methoxy- (oder -methylthio-) -phenylharnstoff,
N-/¯3-Chloro- (oder -Bromo-) -2-methylpropyl_7-N-3-methoxy- (oder -methylthio-) -phenyl-N'-phenyl-harnstoff,
N-/¯3-Chloro- (oder -Bromo-) -2-methylpropyl_7-N-3-nitrophenyl-N'-phenyl-harnstoff,
N-/¯3-Chloro- (oder -Bromo-) -2-methylpropyl_7-N-phenyl-N'-3-nitrophenyl-harnstoff,
N-/¯3-Chloro- (oder -Bromo-) -2-methylpropyl_7-N-3-phenoxyphenyl-N'-phenyl-harnstoff,
N-/¯3-Chloro- (oder -Bromo-) -2-methylpropyl_7-N- phenyl-N'-3-phenoxyphenyl-harnstoff,
N-/¯3-Chloro- (oder -Bromo-) -2-methylpropyl_7-N-3-trifluoromethylphenyl-N'-phenyl-harnstoff,
N-/¯3-Chloro- (oder -Bromo-) -2-methylpropyl_7-N-phenyl-N'-3-trifluoromethylphenyl-harnstoff,
N-/¯3-Chloro- (oder -Bromo-) -2-methylpropyl_7-N-3,4-dichlorophenyl-N'-phenyl-harnstoff,
N-/¯3-Chloro- (oder -Bromo-) -2-methylpropyl_7-N-phenyl-N'-3,4-dichlorophenyl-harnstoff,

N-/¯3-Chloro- (oder -Bromo-) -2-methylpropyl_7-N-3,5-dichloro- (oder -dimethoxy- ) -phenyl-N'-phenyl-harnstoff,

N-/¯3-Chloro- (oder -Bromo-) -2-methylpropyl_7-N-phenyl-N'-3,5- dichloro- (oder -dimethoxy- ) -phenyl-harnstoff,

N-/¯3-Chloro- (oder -Bromo-) -2-methylpropyl_7-N-α-naphthyl-N'-phenyl-harnstoff,

N-/¯3-Chloro- (oder -Bromo-) -2-methylpropyl_7-N-phenyl-N'-α-naphthyl-harnstoff,

N-/¯3-Chloro- (oder -Bromo-) -2-methylpropyl_7-N-2,4,5-trichlorophenyl-N'-phenyl-harnstoff,

N-/¯3-Chloro- (oder -Bromo-) -2-methylpropyl_7-N-phenyl-N'-2,4,5-trichlorophenyl-harnstoff,

N-/¯3-Chloro- (oder -Bromo-) -2-methylpropyl_7-N-2-chlorophenyl-N'-3-chloro- (oder -trifluoromethyl- ) -phenylharnstoff,

N-/¯3-Chloro- (oder -Bromo-) -2-methylpropyl_7-N-3-chloro- (oder -trifluoromethyl- ) -phenyl-N'-2-chloro-phenylharnstoff,

N-/¯3-Chloro- (oder -Bromo-) -2-methylpropyl_7-N-2-tolyl-N'-3-chloro- (oder -methylthio- oder -trifluoromethyl- ) -phenylharnstoff,

N-/¯3-Chloro- (oder -Bromo-) -2-methylpropyl_7-N-3-chloro- (oder -methylthio- oder -trifluoromethyl- ) -phenyl-N'-2-tolylharnstoff,

N-/¯3-Chloro- (oder -Bromo-) -2-methylpropyl_7-N-3,5-xylyl-N'-phenylharnstoff,

N-/¯3-Chloro- (oder -Bromo-) -2-methylpropyl_7-N-phenyl-N'-3,5-xylylharnstoff,

N-/¯3-Chloro- (oder -Bromo-) -2-methylpropyl_7-N-2-methoxyphenyl-N'-3-chloro- (oder -methylthio- oder -trifluoromethyl- ) -phenylharnstoff,

N-[3-Chloro- (oder -Bromo-) -2-methylpropyl]-N-3-chloro- (oder -methylthio- oder -trifluoromethyl- ) -phenyl-N'-2-methoxyphenylharnstoff,

N-[3-Chloro- (oder -Bromo-) -2-methylpropyl]-N-3-tolyl-N'-2-methoxyphenylharnstoff,

N-[3-Chloro- (oder -Bromo-) -2-methylpropyl]-N-2-methoxyphenyl-N'-3-tolylharnstoff,

N-[3-Chloro- (oder -Bromo-) -2-methylpropyl]-N-3-chlorophenyl-N'-3-chloro- (oder -trifluoromethyl- ) -phenylharnstoff,

N-[3-Chloro- (oder -Bromo-) -2-methylpropyl]-N-3-chloro- (oder -trifluoromethyl- ) -phenyl-N'-3-chloro- -phenylharnstoff,

N-[3-Chloro- (oder -Bromo-) -2-methylpropyl]-N-3-tolyl-N'-3-chlorophenylharnstoff,

N-[3-Chloro- (oder -Bromo-) -2-methylpropyl]-N-3-chlorophenyl-N'-3-tolylharnstoff,

N-[3-Chloro- (oder -Bromo-) -2-methylpropyl]-N-2-methoxyphenyl-N'-3,4-dichloro- (oder -3,5-dichloro- ) -phenylharnstoff,

N-[3-Chloro- (oder -Bromo-) -2-methylpropyl]-N-3,4-dichloro- (oder -3,5-dichloro- ) -phenyl-N'-2-methoxyphenylharnstoff,

N-[3-Chloro- (oder -Bromo-) -2-methylpropyl]-N-2-tolyl-N'-3,5-dichlorophenylharnstoff,

N-[3-Chloro- (oder -Bromo-) -2-methylpropyl]-N-3,5-dichlorophenyl-N'-2-tolylharnstoff,

N-[3-Chloro- (oder -Bromo-) -1-methyl- (oder -3-methyl-) -propyl]-N-phenyl-N'-3-chloro- (oder -trifluoromethyl- ) -phenylharnstoff,

N-[3-Chloro- (oder -Bromo-) -1-methyl- (oder -3-methyl-) -propyl]-N-3-chloro- (oder -trifluoromethyl- ) -phenyl-N'-phenylharnstoff,

N-/‾3-Chloro- (oder -Bromo-) -1-methyl- (oder -3-methyl-) -propyl_7-N-phenyl-N'-3,5-dichlorophenylharnstoff und
N-/‾3-Chloro- (oder -Bromo-) -1-methyl- (oder -3-methyl-) -propyl_7-N-3,5-dichlorophenyl-N'-phenylharnstoff.

Bevorzugte Alkalimetallhydroxide der allgemeinen Formel (III), die auch Ausgangsmaterialien in der Reaktion zur Herstellung der Verbindungen der Formel (I) sind, sind Lithiumhydroxid, Natriumhydroxid und Kaliumhydroxid.

Wenn die angegebenen Ausgangsstoffe verwendet werden, wird das Verfahren zur Herstellung der Verbindungen der allgemeinen Formel (I) durch die nachstehende Gleichung erläutert:

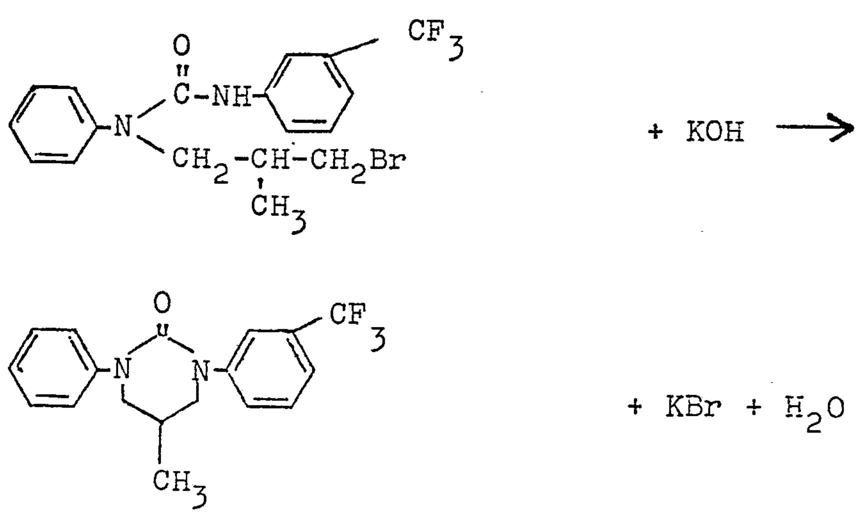


Das Verfahren zur Herstellung der Verbindungen der Formel (I) gemäß der vorliegenden Erfindung wird vorzugsweise in Gegenwart eines Lösungsmittels oder Verdünnungsmittels durchgeführt. Zu diesem Zweck können sämtliche inerten Lösungsmittel und Verdünnungsmittel verwendet werden. Zu diesen zählen insbesondere Wasser, aliphatische, alicyclische und aromatische - gegebenenfalls chlorierte - Kohlenwasserstoffe (wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol), Ether (wie Diethylether, Methylethylether, Di-isopropylether, Dibutylether, Propylenoxid, Dioxan und Tetrahydrofuran), Ketone (wie Aceton, Methylethylketon, Methylisopropylketon und Methylisobutylketon), Nitrile (wie Acetonitril, Propionitril und Acrylnitril), Alkohole (wie Methanol, Ethanol, Isopropanol, Butanol und Ethylenglycol), Ester (wie Ethylacetat und Amylacetat), Säureamide (wie Dimethylformamid und Dimethylacetamid), Sulfone und Sulfoxide (wie Dimethylsulfoxid und Sulfolan) und Basen (wie Pyridin).

Die Reaktionstemperatur kann innerhalb eines beträchtlichen Bereichs verändert werden. Im allgemeinen wird die Reaktion bei einer Temperatur zwischen -20°C und dem Siedepunkt der Reaktionsmischung, vorzugsweise zwischen 0°C und 100°C, durchgeführt.

Das Verfahren gemäß der vorliegenden Erfindung wird vorzugsweise unter dem Druck der Umgebung durchgeführt, obwohl es auch unter erhöhtem oder vermindertem Druck durchgeführt werden kann.

Wie bereits im Vorstehenden erwähnt sind die N,N'-Diaryl-N-halogenoalkylharnstoffe der obigen Formel (II)

neue Verbindungen, die einen weiteren Gegenstand der vorliegenden Erfindung bilden und als Zwischenprodukte auf dem Wege zu den methyl-substituierten Tetrahydro-2-pyrimidinon-Derivaten der vorstehenden Formel (I) wertvoll sind, die eine ausgezeichnete selektive herbizide Wirksamkeit besitzen.

Das weitere Verfahren gemäß der vorliegenden Erfindung zur Herstellung der Ausgangs-Verbindungen der Formel (II) wird durch die folgende Gleichung beschrieben

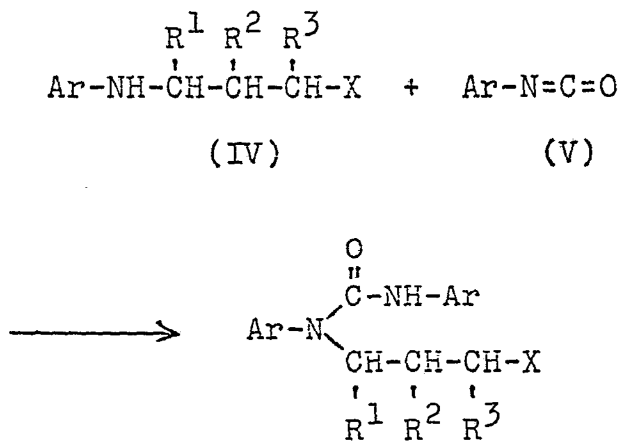

(IV) (V)

(II)

(in der Ar, $R^1$, $R^2$, $R^3$ und X die im Vorstehenden angegebenen Bedeutungen haben).

Beispiele für die Verbindungen der Formel (IV), die zu erwähnen sind, umfassen:
N-/¯3-Chloro- (oder -Bromo-) -2-methylpropyl_7anilin,
N-/¯3-Chloro- (oder -Bromo-) -2-methylpropyl_7-3-fluoro- (oder -chloro- oder -bromo-) -anilin,
N-/¯3-Chloro- (oder -Bromo-) -2-methylpropyl_7-3-methoxy- (oder -methylthio-) -anilin,

N-/¯3-Chloro- (oder -Bromo-) -2-methylpropyl_7-3-nitroanilin,

N-/¯3-Chloro- (oder -Bromo-) -2-methylpropyl_7-3-phenoxyanilin,

N-/¯3-Chloro- (oder -Bromo-) -2-methylpropyl_7-3-trifluoromethylanilin,

N-/¯3-Chloro- (oder -Bromo-) -2-methylpropyl_7-3,4-dichloroanilin,

N-/¯3-Chloro- (oder -Bromo-) -2-methylpropyl_7-3,5-dichloro- (oder -dimethoxy-) -anilin,

N-/¯3-Chloro- (oder -Bromo-) -2-methylpropyl_7- -naphthylamin,

N-/¯3-Chloro- (oder -Bromo-) -2-methylpropyl_7-2,4,5-trichloroanilin,

N-/¯3-Chloro- (oder -Bromo-) -2-methylpropyl_7-2-chloroanilin,

N-/¯3-Chloro- (oder -Bromo-) -2-methylpropyl_7-2-toluidin,

N-/¯3-Chloro- (oder -Bromo-) -2-methylpropyl_7-2-methoxyanilin,

N-/¯3-Chloro- (oder -Bromo-) -2-methylpropyl_7-3-toluidin,

N-/¯3-Chloro- (oder -Bromo-) -1-methyl- (oder -3-methyl-) -propyl_7anilin,

N-/¯3-Chloro- (oder -Bromo-) -1-methyl- (oder -3-methyl-) -propyl_7-3-chloro- (oder -trifluoromethyl-) -anilin,

N-/¯3-Chloro- (oder -Bromo-) -2-methylpropyl_7-3,5-xylidin und

N-/¯3-Chloro- (oder -Bromo-) -1-methyl- (oder -3-methyl-) -propyl_7-3,5-dichloroanilin.

In gleicher Weise umfassen spezielle Beispiele für das Ausgangs-Isocyanat der allgemeinen Formel (V)

Phenylisocyanat,
3-Fluoro- (oder -chloro- oder -bromo-)-phenylisocyanat,
3-Methoxy- (oder -methylthio-) -phenylisocyanat,
3-Nitrophenylisocyanat,
3-Phenoxyphenylisocyanat,
3-Trifluoromethylphenylisocyanat,
3,4-Dichlorophenylisocyanat,
3,5-Dichlorophenylisocyanat,
3,5-Xylylisocyanat,
α-Naphthylisocyanat,
3,5-Dimethoxyphenylisocyanat,
2,4,5-Trichlorophenylisocyanat,
2-Chlorophenylisocyanat,
2-Tolylisocyanat,
2-Methoxyphenylisocyanat und
3-Tolylisocyanat.

Wenn die angegebenen Ausgangsstoffe verwendet werden, wird das Verfahren zur Herstellung der Verbindungen der allgemeinen Formel (III) durch die nachstehende Gleichung erläutert:

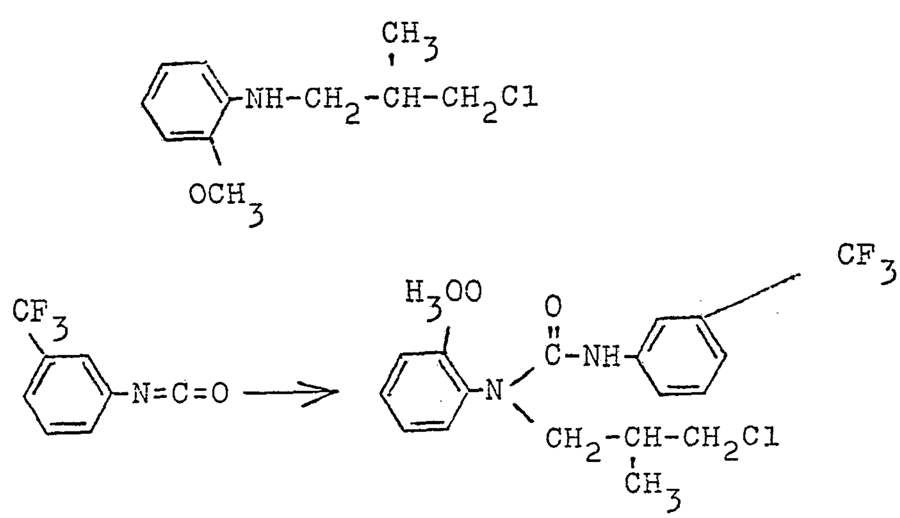

Das Verfahren gemäß der vorliegenden Erfindung zur Herstellung der Verbindungen der Formel (II) wird vorzugsweise in Gegenwart eines Lösungsmittels oder Verdünnungsmittels durchgeführt. Zu diesem Zweck können sämtliche inerten Lösungsmittel und Verdünnungsmittel, die im Vorstehenden für das Verfahren zur Herstellung der Verbindungen der Formel (I) genannt wurden, verwendet werden.

Die Reaktionstemperatur kann innerhalb eines beträchtlichen Bereichs verändert werden. Im allgemeinen wird die Reaktion bei einer Temperatur zwischen -20°C und dem Siedepunkt der Reaktionsmischung, vorzugsweise zwischen 0°C und 100°C, durchgeführt.

Das Verfahren gemäß zur Herstellung der Verbindungen der Formel (II) wird vorzugsweise unter dem Druck der Umgebung durchgeführt, obwohl es auch unter erhöhtem oder vermindertem Druck durchgeführt werden kann.

In einer bevorzugten Ausführungsform des Verfahrens gemäß der vorliegenden Erfindung können die methyl-substituierten Tetrahydropyrimidinon-Derivate der allgemeinen Formel (I) in hohen Ausbeuten und mit hoher Reinheit dadurch hergestellt werden, daß das Ausgangsmaterial der Formel (II), das aus den Verbindungen der allgemeinen Formeln (IV) und (V) durch das Verfahren zur Herstellung dieses Zwischenprodukts erhalten wurde, eingesetzt wird, ohne daß die als Zwischenprodukt anfallende Verbindung der Formel (II) isoliert zu werden braucht.

Die Ausführungsform wird durch die folgende Gleichung dargestellt:



BAD ORIGINAL

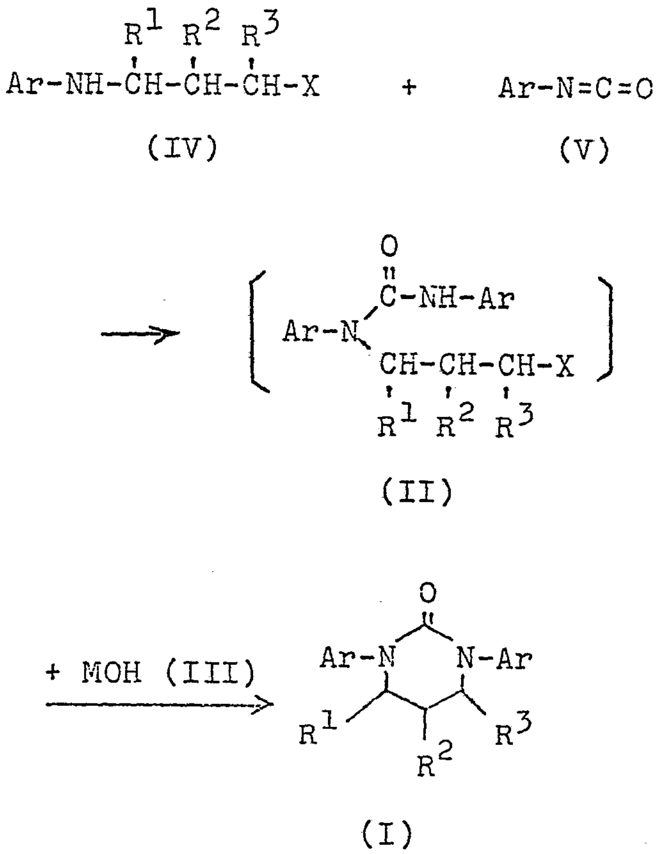


(IV) (V)

(II)

(I)

Im vorstehenden Reaktionsschema haben Ar, $R^1$, $R^2$, $R^3$ und X die im Vorstehenden angegebenen Bedeutungen.

Wenn die bezeichneten Ausgangsstoffe eingesetzt werden, wird diese Ausführungsform weiter durch die folgende Gleichung veranschaulicht:

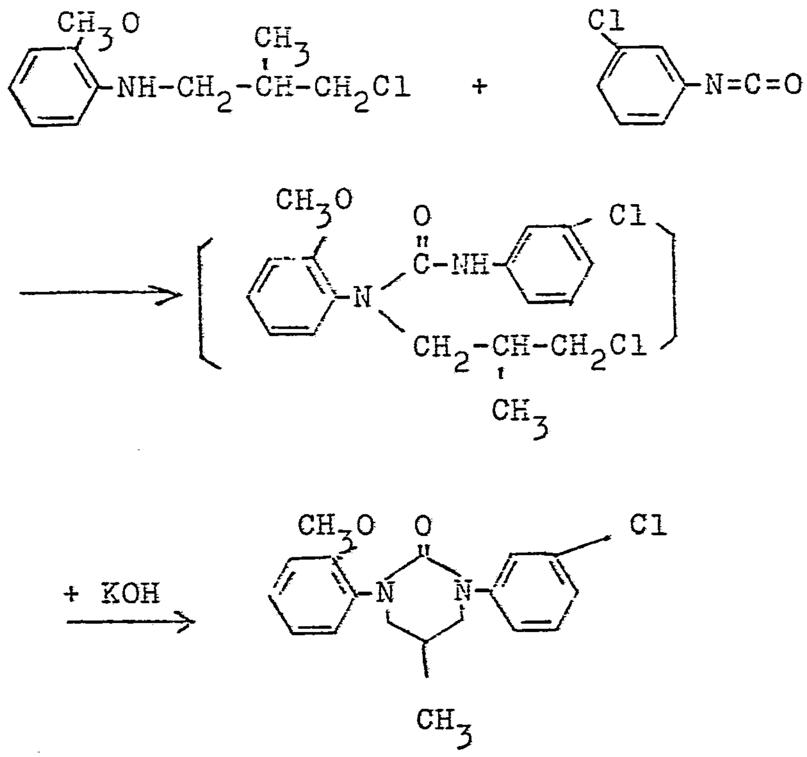


Die Reaktion dieser Ausführungsform wird unter Verwendung der gleichen inerten Lösungsmittel oder Verdünnungsmittel durchgeführt, die im Vorstehenden genannt wurden, und sie kann in wirkungsvoller Weise in Anwesenheit eines Katalysators wie Tetrabutylammoniumbromid durchgeführt werden. Die in dieser Ausführungsform angewandten Temperaturen und Drücke entsprechen gleichfalls den im Vorstehenden angegebenen.

Da die Verbindungen der Formel (I) gemäß der vorliegenden Erfindung eine geringe Toxizität gegenüber warmblütigen Tieren und die bereits erwähnte ausgezeichnete Selektivität besitzen, eignen sich diese Verbindungen in besonderem Maße für die Unkrautbekämpfung. Insbesondere zeigen die erfindungsgemäßen Verbindungen der Formel (I) eine sehr gute Selektivität gegenüber Nutzpflanzen zusammen mit einer hervorragenden Wirksamkeit der Unkrautbekämpfung, wenn sie als Mittel bei der Behandlung vor und nach dem Auflaufen beim Anbau von Baumwolle und Reis eingesetzt werden. Die Verbindungen der Formel (I) gemäß der Erfindung besitzen eine hohe Sicherheit gegenüber den Nutzpflanzen, eine herausragende herbizide Aktivität sowie ein breites herbizides Spektrum.

Unter "Unkräutern" im weitesten Sinne sind solche Pflanzen zu verstehen, die an Stellen wachsen, wo sie nicht erwünscht sind.

Die aktiven Verbindungen gemäß der vorliegenden Erfindung sind ausgezeichnet in bezug auf ihre Sicherheit, beweisen eine überlegene herbizide Wirksamkeit und weisen breite herbizide Spektren auf.

Die Wirkstoffe gemäß der vorliegenden Erfindung können beispielsweise zur Bekämpfung der folgenden Pflanzen verwendet werden:

Dikotyledonen-Unkräuter der Gattungen
Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Rorippa, Rotala, Lindernia, Lamium, Veronica, Abutilon, Emex, Datura, Viola, Galeopsis, Papaver, Centaurea und Solanum; und
Monokotyledonen-Unkräuter der Gattungen
Echinochloa, Setaria, Panicum, Digitaria, Phleum,



BAD ORIGINAL

Poa, Festuca, Eleusine, Brachiaria, Lolium, Bromus, Avena, Cyperus, Sorghum, Agropyron, Cynodon, Monocharia, Fimbristylis, Sagittaria, Eleocharis, Scirpus, Paspalum, Ischaemum, Spenoclea, Dactyloctenium, Agrostis, Alopecurus und Apera.

Die aktiven Verbindungen gemäß der vorliegenden Erfindung können beispielsweise als selektive Herbizide in den folgenden Kulturen verwendet werden:

Dikotyledonen-Kulturen der Gattungen
Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucurbita; und
Monokotyledonen-Kulturen der Gattungen
Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern umfaßt auch andere Pflanzen in gleicher Weise.

In Abhängigkeit von den Konzentrationen können die Verbindungen zur totalen Bekämpfung von Unkräutern, beispielsweise auf Industriegelände und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Tee-



BAD ORIGINAL

plantagen, Gummiplantagen, Ölpalmenplantagen, Kakaoplantagen, Weichfruchtplantagen und Hopfenfeldern, sowie zur selektiven Unkrautbekämpfung in Jahreskulturen verwendet werden.

Die Verbindungen gemäß der vorliegenden Erfindung zeigen eine überlegene selektive Bekämpfungswirkung. Beispielsweise entfalten sie herbizide Wirkung gegen Unkräuter auf Reisfeldern wie die nachstehend aufgeführten, ohne daß sie irgendeine schädliche Wirkung auf die Reispflanzen ausüben:

Die Dikotyledonen-Unkräuter

Rotala indica Koehne,
Lindernia procumbens Philcox,
Ludwiga prostrata Roxburgh,
Potamogeton distinctus A. Bennett und
Elatine triandra Schkuhr

sowie

die Monokotyledonen-Unkräuter

Echinochloa crus-galli P. Beauvois var.,
Monochoria vaginalis Presl,
Eleocharis acicularis L.,
Eleocharis kuroguwai Ohwi,
Cyperus difformis L.,
Cyperus serotinus Rottboell,
Sagittaria pygmaea Miquel,
Alisma canaliculatum A. Braun et Bouché und
Scirpus juncoides Roxburgh var..

Die Anwendbarkeit der Wirkstoffe der Formel (I) gemäß der Erfindung ist nicht auf Unkräuter in unter Wasser stehenden Reisfeldern und höher gelegenen landwirtschaftlichen Anbauflächen beschränkt. Sie sind eben-



BAD ORIGINAL

falls wirksam gegen Schad-Unkräuter in zur Matten-Herstellung dienenden Binsen (Juncus effusus Linnaeus var. decipiens Buchanan) und gegen Unkräuter auf Land, das zeitweise brach liegt.

Die Wirkstoffkombinationen können umgewandelt werden in übliche Präparate wie Lösungen, Emulsionen, Suspensionen, Pulver, Stäubemittel, Schäume, Pasten, lösliche Pulver, Granulate, Aerosole, Suspensions-Emulsions-Konzentrate, Pulver zur Behandlung von Saatgut, mit dem Wirkstoff getränkte natürliche oder synthetische Materialien, sehr feine Kapseln in polymeren Substanzen, Überzugsmassen zur Anwendung auf Saatgut sowie Präparate, die in Verbrennungsgeräten eingesetzt werden, wie Räucherpatronen, Räucherdosen, Räucherschlangen sowie auch Präparate für die Kalt- oder Warm-Vernebelung nach dem ULV(ultra low volume)-Verfahren.

Diese Präparate können in bekannter Weise hergestellt werden, beispielsweise durch Vermischen der Wirkstoffkombinationen mit Streckmitteln, d.h. flüssigen oder festen Verdünnungsmitteln oder Trägern, gegebenenfalls unter Verwendung von oberflächenaktiven Mitteln, d.h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger, insbesondere als Lösungsmittel, eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische oder ali-



BAD ORIGINAL

cyclische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon, oder stark polare Lösungsmittel wie Dimethylformamid oder Dimethylsulfoxid, sowie auch Wasser.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen oder organischen Mehlen und Granulat aus organischem Material wie Sägemehl, Kokosnußschalen, Maiskolben und Tabakstengeln verwendet werden.

Als Emulgatoren und/oder Schaumbildner können nichtionische oder anionische Emulgatoren wie Polyoxyethylen-Fettsäureester, Polyoxyethylen-Fettalkoholether, beispielsweise Alkylarylpolyglycolether, Alkylsulfonate, Alkylsulfate und Arylsulfonate sowie Hydrolyseprodukte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulat oder Latices, z.B. Gummiarabicum, Polyvinylalkohol und Polyvinylacetat, können in den Formulierungen verwendet werden.



BAD ORIGINAL

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Farbstoffe und Metallphthalocyanin-Farbstoffe, und Spurennährstoffe wie Salze von Eisen, Mangan, Bor, Kupfer, Cobalt, Molybdän und Zink, zu verwenden.

Die Präparate enthalten im allgemeinen von 0,001 bis 100 Gewichts-%, vorzugsweise von 0,005 bis 95 Gewichts-% der Wirkstoffe.

Die Wirkstoffe gemäß der vorliegenden Erfindung können zur Unkrautbekämpfung als solche oder in Form ihrer Präparate auch in Gemischen mit bekannten Herbiziden angewandt werden; Fertigpräparate oder das Tankmischverfahren kommen als Möglichkeiten hierfür in Betracht. Ein Einsatz von Mischungen mit anderen aktiven Verbindungen wie Fungiziden, Bakteriziden, Insektiziden, Akariziden, Nematoziden, vogelabweisenden Mitteln, Wachstumsfaktoren, Pflanzennährstoffen sowie Mitteln zur Verbesserung der Bodenstruktur ist ebenfalls möglich.

Die Wirkstoffe können als solche, in Form von Präparaten oder in Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie können in üblicher Weise angewandt werden, beispielsweise durch Bewässern, Sprühen, Zerstäuben, Streuen oder Stäuben.



BAD ORIGINAL

Die aktiven Verbindungen gemäß der vorliegenden Erfindung können entweder vor oder nach dem Auflaufen der Pflanzen zur Anwendung gebracht werden. Vorzugsweise werden sie vor dem Auflaufen der Pflanzen, d.h. mittels der Vorauflauf-Behandlung, angewandt. Sie können auch vor der Einsaat in den Boden eingearbeitet werden.

Weiterhin ist es auch möglich, die Wirkstoffe mittels des ULV-Verfahrens (ultra-low-volume method) zur Anwendung zu bringen, wodurch es möglich wird, die Verbindungen in einer Konzentration bis zu 100 Gew.-% einzusetzen.

Im praktischen Einsatz beträgt der Gehalt der Wirkstoffe in den verschiedenen Anwendungsformen oder gebrauchsfertigen Präparaten im allgemeinen von 0,01 bis 95 Gewichts-%, vorzugsweise von 0,05 bis 60 Gewichts-%.

Die flächenbezogenen Aufwandsmengen der Wirkstoffkombinationen betragen im allgemeinen 0,5 bis 5 kg/ha, vorzugsweise 1,0 bis 4,0 kg/ha. In besonderen Fällen ist es jedoch möglich oder manchmal sogar notwendig, auch eine Menge ober- oder unterhalb des genannten Bereichs aufzuwenden.

Die vorliegende Erfindung umfaßt weiter ein herbizides Mittel, das als Wirkstoff eine Verbindung gemäß der vorliegenden Erfindung im Gemisch mit einem festen Verdünnungsmittel oder Träger oder im Gemisch mit einem ein oberflächenaktives Mittel enthaltenden flüssigen Verdünnungsmittel oder Träger enthält.



BAD ORIGINAL

Die vorliegende Erfindung umfaßt weiter ein Verfahren zur Unkrautbekämpfung, das dadurch gekennzeichnet ist, daß eine Verbindung gemäß der vorliegenden Erfindung allein oder in Form eines Präparats, das als Wirkstoff eine Verbindung gemäß der vorliegenden Erfindung im Gemisch mit einem Verdünnungsmittel oder Träger enthält, auf die Unkräuter oder deren Lebensraum aufgebracht wird.

Die vorliegende Erfindung ist ferner auf Nutzpflanzen gerichtet, die dadurch gegen Schäden durch Unkräuter geschützt sind, daß sie auf Flächen angebaut werden, auf die unmittelbar vor und/oder während der Zeit des Anbauens eine Verbindung gemäß der vorliegenden Erfindung allein oder im Gemisch mit einem Verdünnungsmittel oder Träger aufgebracht wurde.

Es wird ersichtlich werden, daß die üblichen Methoden der Erzeugung geernteter Nutzpflanzen durch die vorliegende Erfindung verbessert werden können.

Die Zusammensetzungen gemäß der vorliegenden Erfindung werden durch die folgenden Beispiele (i) bis (vi) erläutert. Hierin werden die erfindungsgemäßen Verbindungen durch die (in Klammer angegebenen) Zahlen der entsprechenden Herstellungsbeispiele bezeichnet.



BAD ORIGINAL

Beispiel (i)

15 Gew.-Teile der Verbindung (1), 80 Gew.-Teile eines Gemisches (1:5) aus pulvriger Diatomeenerde und Tonpulver, 2 Gew.-Teile Natriumalkylbenzolsulfonat und 3 Teile Natriumalkylnaphthalinsulfonat/Formaldehyd-Kondensat wurden pulverisiert und zu einem benetzbaren Pulver vermischt. Dieses wurde vor dem Sprühen mit Wasser verdünnt.

Beispiel (ii)

30 Gew.-Teile der Verbindung (2), 55 Gew.-Teile Xylol, 8 Gew.-Teile Polyoxyethylen-alkylphenylether und 7 Gew.-Teile Calciumalkylbenzolsulfonat wurden unter Rühren miteinander vermischt, wodurch ein emulgierbares Konzentrat hergestellt wurde. Dieses wurde vor dem Sprühen mit Wasser verdünnt.

Beispiel (iii)

2 Gew.-Teile der Verbindung (3) und 98 Teile Tonpulver wurden pulverisiert und gemischt, wodurch ein Stäubemittel hergestellt wurde.

Beispiel (iv)

1,15 Gew.-Teile der Verbindung (4), 0,5 Teile Isopropylhydrogenphosphat und 98 Gew.-Teile Tonpulver wurden pulverisiert und gemischt, wodurch ein Stäubemittel hergestellt wurde.



BAD ORIGINAL

Beispiel (v)

25 Gew.-Teile Wasser wurden zu einer Mischung aus 10 Gew.-Teilen der Verbindung (5), 30 Gew.-Teilen Bentonit (Montmorillonit), 58 Gew.-Teilen Talkum und 2 Gew.-Teilen Ligninsulfonat zugesetzt, und das Gemisch wurde gut geknetet. Die Mischung wurde mittels eines Extruder-Granulators zu einem Granulat mit einer Korngröße von 0,43 bis 2,0 mm (10 bis 40 mesh) verarbeitet und bei 40°C bis 50°C getrocknet, wodurch ein Granulat hergestellt wurde. Dieses wurde durch Streuen aufgebracht.

Beispiel (vi)

Ein Drehmischer wurde mit 95 Gew.-Teilen Tonmineral-Teilchen mit einer Teilchengrößen-Verteilung im Bereich von 0,2 bis 2 mm beschickt, und unter Drehen des Mischers wurden 5 Gew.-Teile der Verbindung (7) gelöst in einem organischen Lösungsmittel zur gleichmäßigen Benetzung auf die Teilchen aufgesprüht. Die Teilchen wurden dann bei 40°C bis 50°C getrocknet, wodurch ein Granulat gebildet wurde. Das Granulat wurde durch Streuen aufgebracht.

Die herbizide Wirkung der Verbindungen gemäß der vorliegenden Erfindung wird durch die folgenden Biotest-Beispiele aufgezeigt.

In diesen Beispielen werden die erfindungsgemäßen Verbindungen durch die (in Klammer angegebenen) Zahlen der entsprechenden Herstellungsbeispiele bezeichnet.



BAD ORIGINAL

Die bekannten Vergleichsverbindungen werden wie folgt identifiziert:

(A) = 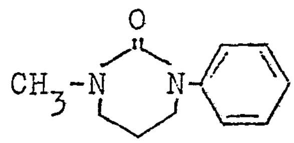 beschrieben in Chem.Abstracts 57, 9860a (1962);

(B) = 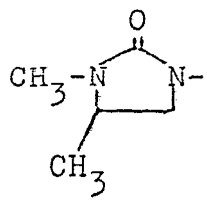 ebenfalls beschrieben in Chem.Abstracts 57, 9860a (1962);

## Beispiel A

Test zur Bestimmung der herbiziden Wirkung gegen Unkräuter auf höher gelegenen Feldern (Topf-Test)

---

Lösungsmittel: 5 Gew.-Teile Aceton
Emulgator: 1 Gew.-Teil Benzyloxypolyglycolether

Zur Herstellung von geeigneten Wirkstoff-Präparaten wurde 1 Gew.-Teil der betreffenden aktiven Verbindung mit der angegebenen Menge Lösungsmittel vermischt, die angegebene Menge Emulgator wurde zugesetzt, und die erhaltene Mischung wurde mit Wasser auf die gewünschte Konzentration verdünnt.



BAD ORIGINAL

Kunststoff-Töpfe (5 $dm^2$; 25 cm x 20 cm x 10 cm) wurden jeweils mit 2,5 l Schwemmland-Boden gefüllt, der durch ein Sieb der Maschenweite 0,5 cm x 0,5 cm gesiebt worden war. Baumwoll-Samen (Varietät: Coker 310) wurde in den Boden in einer Tiefe von 1 cm eingesät, und der gleiche Boden wie im Vorstehenden, der Samen von Digitaria adscendens Henr., Echinochloa crus-galli, Amaranthus retroflexus L., Chenopodium album Linnaeus und Portulaca oleracea Linnaeus enthielt, wurde über den Boden in den Töpfen gegeben. Das Präparat der Chemikalie wurde gleichmäßig auf die Oberflächenschicht jedes der Test-Töpfe in vorher festgelegter Dosierung aufgesprüht.

Die herbizide Wirkung wurde 20 Tage nach der Behandlung geprüft, und der Grad der Phytotoxizität wurde jeweils 20 Tage, 40 Tage und 60 Tage nach der Behandlung geprüft. Die Ergebnisse wurden mit Hilfe einer Skala von 0 bis 10 nach dem folgenden Maßstab bewertet.

Die herbizide Wirkung wurde aufgrund der Unkrautvernichtungsrate im Vergleich zu unbehandelten Kontrollpflanzen durch die Bewertung mit den Zahlen 0 bis 10 wie folgt bezeichnet:

| Bewertung | Unkrautvernichtungsrate (bezogen auf die unbehandelten Kontrollpflanzen) |
|---|---|
| 10 | 100 % (vollständig verdorrt) |
| 9 | mindestens 90 %, jedoch weniger als 100 % |
| 8 | mindestens 80 %, jedoch weniger als 90 % |
| 7 | mindestens 70 %, jedoch weniger als 80 % |
| 6 | mindestens 60 %, jedoch weniger als 70 % |
| 5 | mindestens 50 %, jedoch weniger als 60 % |
| 4 | mindestens 40 %, jedoch weniger als 50 % |
| 3 | mindestens 30 %, jedoch weniger als 40 % |
| 2 | mindestens 20 %, jedoch weniger als 30 % |
| 1 | mindestens 10 %, jedoch weniger als 20 % |
| 0 | weniger als 10 % (keine herbizide Wirkung) |

Die Phytotoxizität gegenüber den Baumwollpflanzen wurde aufgrund der Phytotoxizitätsrate im Vergleich zu den unbehandelten Kontrollpflanzen mit den Zahlen 0 bis 10 wie folgt bezeichnet:

| Bewertung | Phytotoxizitätsrate (bezogen auf die unbehandelten Kontrollpflanzen) |
|---|---|
| 10 | mindestens 90 % (Ausrottung) |
| 9 | mindestens 80 %, jedoch weniger als 90 % |
| 8 | mindestens 70 %, jedoch weniger als 80 % |
| 7 | mindestens 60 %, jedoch weniger als 70 % |
| 6 | mindestens 50 %, jedoch weniger als 60 % |
| 5 | mindestens 40 %, jedoch weniger als 50 % |
| 4 | mindestens 30 %, jedoch weniger als 40 % |
| 3 | mindestens 20 %, jedoch weniger als 30 % |
| 2 | mindestens 10 %, jedoch weniger als 20 % |
| 1 | mehr als 0 %, jedoch weniger als 10 % |
| 0 | 0 % (keine Phytotoxizität) |

Die Testergebnisse sind in der Tabelle 1 aufgeführt.



BAD ORIGINAL

Tabelle 1

| Verbindung Nr. | Wirkstoff-Menge kg/ha | Herbizide Wirkung Unkräuter* D. | E. | A. | C. | P. | Phytotoxizität Baumwolle 20 Tage | 40 Tage | 60 Tage |
|---|---|---|---|---|---|---|---|---|---|
| (1) | 4,0 | 10 | 10 | 10 | 10 | 10 | 0 | 0 | 0 |
| (2) | 4,0 | 10 | 10 | 10 | 10 | 10 | 0 | 0 | 0 |
| (3) | 4,0 | 10 | 10 | 10 | 10 | 10 | 0 | 0 | 0 |
| (4) | 4,0 | 10 | 10 | 10 | 10 | 10 | 0 | 0 | 0 |
| (5) | 4,0 | 10 | 10 | 10 | 10 | 10 | 0 | 0 | 0 |
| (6) | 4,0 | 10 | 10 | 10 | 10 | 10 | 0 | 0 | 0 |
| (7) | 4,0 | 10 | 10 | 10 | 10 | 10 | 0 | 0 | 0 |
| (8) | 4,0 | 10 | 10 | 10 | 10 | 10 | 0 | 0 | 0 |
| (9) | 4,0 | 10 | 10 | 10 | 10 | 10 | 0 | 0 | 0 |
| (10) | 4,0 | 10 | 10 | 10 | 10 | 10 | 0 | 0 | 0 |
| (11) | 4,0 | 10 | 10 | 10 | 10 | 10 | 0 | 0 | 0 |
| (12) | 4,0 | 10 | 10 | 10 | 10 | 10 | 0 | 0 | 0 |
| (13) | 4,0 | 10 | 10 | 10 | 10 | 10 | 0 | 0 | 0 |
| (14) | 4,0 | 10 | 10 | 10 | 10 | 10 | 0 | 0 | 0 |
| (15) | 4,0 | 10 | 10 | 10 | 10 | 10 | 0 | 0 | 0 |
| (16) | 4,0 | 10 | 10 | 10 | 10 | 10 | 0 | 0 | 0 |
| (17) | 4,0 | 10 | 10 | 10 | 10 | 10 | 0 | 0 | 0 |
| (18) | 4,0 | 10 | 10 | 10 | 10 | 10 | 0 | 0 | 0 |

* In Tabelle 1 bezeichnen die Abkürzungen

D. Digitaria adscendens Henr.,

E. Echinochloa crus-galli,

A. Amaranthus retroflexus L.,

C. Chenopodium album Linnaeus und

P. Portulaca oleracea Linnaeus.

Tabelle 1 - Fortsetzung

| Verbindung Nr. | Wirkstoff-Menge kg/ha | Herbizide Wirkung Unkräuter* D. | E. | A. | C. | P. | Phytotoxizität Baumwolle 20 Tage | 40 Tage | 60 Tage |
|---|---|---|---|---|---|---|---|---|---|
| (19) | 4,0 | 10 | 10 | 10 | 10 | 10 | 0 | 0 | 0 |
| (20) | 4,0 | 10 | 10 | 10 | 10 | 10 | 0 | 0 | 0 |
| (21) | 4,0 | 10 | 10 | 10 | 10 | 10 | 0 | 0 | 0 |
| (22) | 4,0 | 10 | 10 | 10 | 10 | 10 | 0 | 0 | 0 |
| (23) | 4,0 | 10 | 10 | 10 | 10 | 10 | 0 | 0 | 0 |
| (24) | 4,0 | 10 | 10 | 10 | 10 | 10 | 0 | 0 | 0 |
| (25) | 4,0 | 10 | 10 | 10 | 10 | 10 | 0 | 0 | 0 |
| (26) | 4,0 | 10 | 10 | 10 | 10 | 10 | 0 | 0 | 0 |
| (27) | 4,0 | 10 | 10 | 10 | 10 | 10 | 0 | 0 | 0 |
| (28) | 4,0 | 10 | 10 | 10 | 10 | 10 | 0 | 0 | 0 |
| (29) | 4,0 | 10 | 10 | 10 | 10 | 10 | 0 | 0 | 0 |
| (30) | 4,0 | 10 | 10 | 10 | 10 | 10 | 0 | 0 | 0 |
| Vergleich | | | | | | | | | |
| (A) | 4,0 | 1 | 1 | 0 | 0 | 1 | 0 | 0 | 0 |
| (B) | 4,0 | 0 | 0 | 0 | 0 | 0 | 0 | 0 | 0 |

* In Tabelle 1 bezeichnen die Abkürzungen

D. Digitaria adscendens Henr.,

E. Echinochloa crus-galli,

A. Amaranthus retroflexus L.,

C. Chenopodium album Linnaeus und

P. Portulaca oleracea Linnaeus.

Beispiel B

Test zur Bestimmung der Wirkung gegen im Wasser wachsende Reisfeld-Unkräuter durch Behandlung des Bodens sowie der Stengel und Blätter unter Bewässerungs-Bedingungen (Topf-Test):

---

Reis-Kulturboden wurde in Wagner-Töpfe (2 $dm^2$) gefüllt, und zwei Reis-Setzlinge (Varietät: Kinnampu) im 2- bis 3-Blattstadium (Pflanzenhöhe etwa 10 cm) wurden in jeden Topf umgepflanzt. Samen von breitblättrigen Unkräutern, Echinochloa crus-galli, Cyperus sp., Monochoria vaginalis Presl und Scirpus juncoides Roxburgh var. wurden eingesät, und kleine Stücke von Eleocharis acicularis L. und Knollen von Cyperus serotinus Rottboell und Sagittaria pygmaea Miquel wurden in den Boden eingesetzt. Die Töpfe wurden in feuchtem Zustand gehalten. Als die Pflänzchen von Echinochloa crus-galli etwa bis zum 2-Blatt-Stadium gewachsen war (etwa 7 bis 9 Tage nach der Einsaat), wurden die Töpfe mit Wasser bis zu einer Tiefe von etwa 6 cm überflutet. Jedes der in gleicher Weise wie in Beispiel A hergestellten Präparate wurde mittels einer Pipette zugegeben. Nach der Behandlung wurde das Wasser im Laufe von zwei Tagen mit einer Geschwindigkeit von 2 bis 3 cm pro Tag aus den Töpfen heraussickern gelassen. Danach wurden die Töpfe bis zu einer Wassertiefe von etwa 3 cm überflutet. In der vierten Woche nach der Behandlung mit der Chemikalie wurden die herbizide Wirkung und der Grad der Phytotoxizität mit Hilfe einer Skala von 0 bis 10 in gleicher Weise wie in Beispiel A bewertet. Die Ergebnisse sind in der folgenden Tabelle 2 dargestellt.



BAD ORIGINAL

Tabelle 2

| Verbindung | Wirkstoff-Menge kg/ha | Herbizide Wirkung Unkräuter A | B | C | D | E | F | G | H | Phytotoxität gegen Reis |
|---|---|---|---|---|---|---|---|---|---|---|
| (6) | 2,0 | 10 | 10 | 10 | 10 | 10 | 10 | 10 | 10 | 0 |
| (17) | 2,0 | 10 | 10 | 10 | 10 | 10 | 10 | 10 | 10 | 0 |
| Vergleich | | | | | | | | | | |
| (A) | 4,0 | 1 | 0 | 1 | 0 | 1 | 3 | 0 | 2 | 0 |
| (B) | 4,0 | 1 | 0 | 0 | 0 | 0 | 0 | 0 | 0 | 0 |

Anmerkung:

Die Symbole A bis H bezeichnen die nachstehenden Unkräuter:

A: Echinochloa crus-galli
B: Eleocharis acicularis
C: Cyperus sp.
D: Scirpus juncoides
E: Monochoria vaginalis Presl
F: Breitblättrige Unkräuter (wie Lindernia procumbens, Rotala indica und Elatine triandra)
G: Cyperus serotinus
H: Sagittaria pygmaea.

Die folgenden Beispiele 1 bis 30 beschreiben die Herstellung der methyl-substituierten Tetrahydro-2-Pyrimidinon-Derivate der allgemeinen Formel (I) der vorliegenden Erfindung, während die Beispiele 31 bis 62 die Herstellung der neuen Zwischenprodukte der allgemeinen Formel (II) gemäß der vorliegenden Erfindung erläutern.

## Herstellungsbeispiele

### Beispiel 1

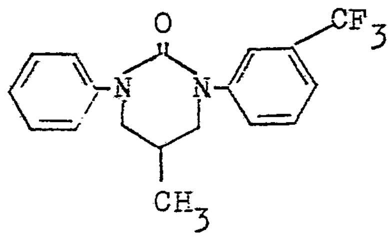

(1)

37 g N-(3-Chloro-2-methylpropyl)-N-phenyl-N'-3-trifluoromethylphenylharnstoff wurden in 150 ml Ethanol gelöst, und 56 g einer ethanolischen Lösung mit 20 Gew.-% Kaliumhydroxid wurden hinzugefügt. Sie reagierten exotherm miteinander. Zur Vervollständigung der Reaktion wurde die Mischung gerührt und 2 h unter Rückfluß erhitzt. Dann wurde das Ethanol unter vermindertem Druck abdestilliert, und dem Rückstand wurde Wasser zugesetzt. Das angestrebte Produkt fiel in Form von Kristallen aus. Die Umkristallisation aus Methanol lieferte 27 g des gewünschten 5-Methyl-1-phenyl-3-(3-trifluoromethylphenyl)-tetrahydro-2-pyrimidinons in Form farbloser Kristalle; Schmp. 121-123°C.



BAD ORIGINAL

Beispiel 2

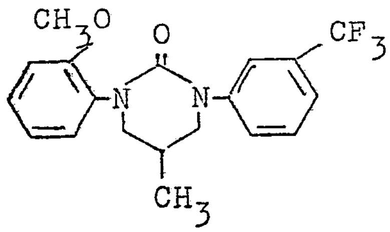

(2)

40 g N-(3-Chloro-2-methylpropyl)-N-2-methoxyphenyl-N'-3-trifluoromethylphenylharnstoff wurden in 150 ml Isopropanol gelöst, und 20 g einer wäßrigen Lösung mit 50 Gew.-% Kaliumhydroxid wurden zu der Lösung bei 30°C bis 35°C unter gutem Rühren hinzugegeben. Die Reaktionsmischung wurde dann 2 h bei 65°C bis 75°C gerührt, und danach wurde das Isopropanol unter vermindertem Druck abdestilliert. Zu dem Rückstand wurden 100 ml Toluol gegeben, und die Mischung wurde mit HCl von 1 Gew.-% und dann mit Wasser gewaschen. Nach dem Entwässern wurde das Toluol unter vermindertem Druck abdestilliert, wonach 26,6 g des gewünschten 1-(2-Methoxyphenyl)-5-methyl-3-(3-trifluoromethylphenyl)-tetrahydro-2-pyrimidinons als viskose, blaßgelbe Flüssigkeit erhalten wurden; $n_D^{19}$ 1,5398.

Die Verbindungen der folgenden Beispiele 3 bis 5 wurden im wesentlichen in der gleichen Weise wie in den vorhergehenden Beispielen 1 und 2 beschrieben hergestellt.

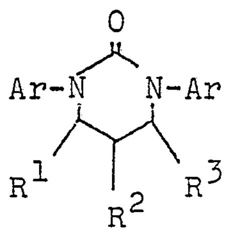

| Beispiel Nr. | Ar | | $R^1$ | $R^2$ | $R^3$ | Schmp. (°C) /Brechungs-Index $n_D^{20}$ |
|---|---|---|---|---|---|---|
| 3 | Phenyl | 3-Chlorophenyl | H | $-CH_3$ | H | 124-126 |
| 4 | 2-Chlorophenyl | 3-Chlorophenyl | H | $-CH_3$ | H | 1,5936 |
| 5 | 2-Chlorophenyl | 3-Trifluoromethylphenyl | H | $-CH_3$ | H | 61-64 |

Beispiel 6

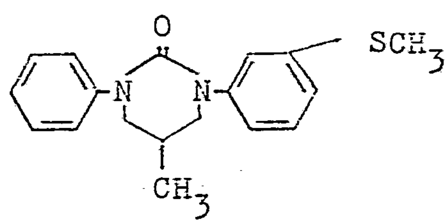

(6)

18,4 g N-(3-Chloro-2-methylpropyl)anilin wurden in 120 ml Toluol gelöst, und 16,5 g 3-Methylthiophenylisocyanat wurden bei Raumtemperatur hinzugefügt. Dann wurde die Lösung 1 h bei 40°C bis 50°C gerührt. Eine katalytische Menge (0,2 g) Tetrabutylammoniumbromid wurde danach zugegeben. Während die Temperatur der Mischung auf 30°C bis 40°C gehalten wurde, wurden dieser 34 g einer wäßrigen Kaliumhydroxid-Lösung mit 50 Gew.-% tropfenweise zugesetzt. Nach der Zugabe wurde die Mischung zur Vervollständigung der Reaktion 1 h bei 40°C

bis 50°C gerührt. Die Reaktionsmischung wurde auf Raumtemperatur abgekühlt. Die Toluol-Schicht wurde abgetrennt und mit HCl von 1 Gew.-% und Wasser in dieser Reihenfolge gewaschen. Nach dem Entwässern wurde das Toluol unter vermindertem Druck abdestilliert, wonach 20,3 g des gewünschten 5-Methyl-1-(3-methylthiophenyl)-3-phenyl-tetrahydro-2-pyrimidinons in Form einer viskosen, blaßgelben Flüssigkeit erhalten wurden; $n_D^{20}$ 1,6194.

Die Verbindungen der folgenden Beispiele 7 bis 30 wurden im wesentlichen wie in dem vorhergehenden Beispiel 6 beschrieben hergestellt.



BAD ORIGINAL

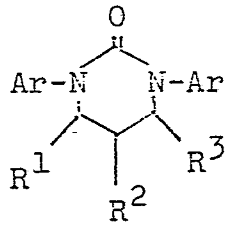

| Beispiel Nr. | | Ar | $R^1$ | $R^2$ | $R^3$ | Schmp. (°C) /Brechungs-Index |
|---|---|---|---|---|---|---|
| 7 | 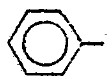 | 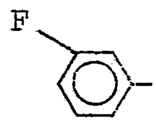 | H | $-CH_3$ | H | 99-101 |
| 8 | " | 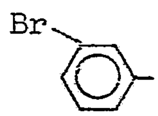 | H | $-CH_3$ | H | 118-121 |
| 9 | " | 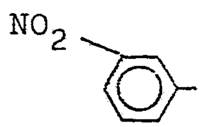 | H | $-CH_3$ | H | 106-108 |
| 10 | " | 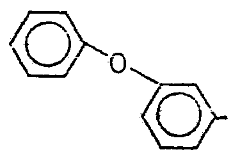 | H | $-CH_3$ | H | $n_D^{19}$ 1.6045 |
| 11 | " | 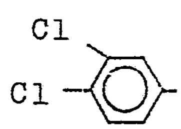 | H | $-CH_3$ | H | 116.5-118.5 |
| 12 | " | 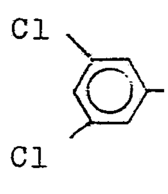 | H | $-CH_3$ | H | 122-125 |
| 13 | " | 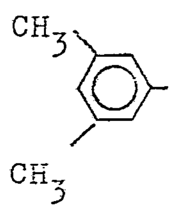 | H | $-CH_3$ | H | $n_D^{20}$ 1.5849 |

| Beispiel Nr. | | Ar | $R^1$ | $R^2$ | $R^3$ | Schmp. (°C) /Brechungs-Index |
|---|---|---|---|---|---|---|
| 14 | 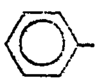 | 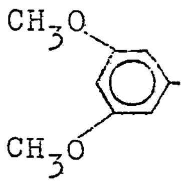 | H | $-CH_3$ | H | $n_D^{19}$ 1.5865 |
| 15 | " | 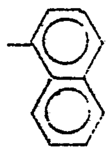 | H | $-CH_3$ | H | $n_D^{20}$ 1.6266 |
| 16 | " | 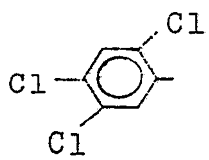 | H | $-CH_3$ | H | 141-143 |
| 17 | " | 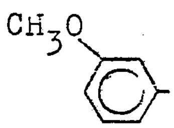 | H | $-CH_3$ | H | 94-97 |
| 18 | 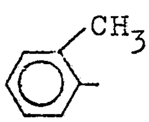 | 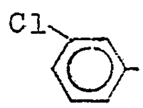 | H | $-CH_3$ | H | $n_D^{20}$ 1.5900 |
| 19 | " | 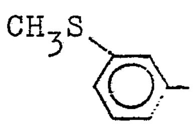 | H | $-CH_3$ | H | $n_D^{20}$ 1.5976 |
| 20 | " | 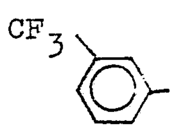 | H | $-CH_3$ | H | 141.5-143 |
| 21 | 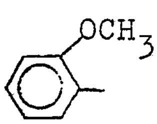 | 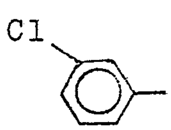 | H | $-CH_3$ | H | 91-93 |
| 22 | " | 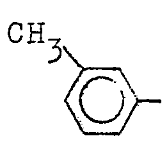 | H | $-CH_3$ | H | 104-106 |

| Beispiel Nr. | Ar | | $R^1$ | $R^2$ | $R^3$ | Schmp. (°C) /Brechungs-Index |
|---|---|---|---|---|---|---|
| 23 | 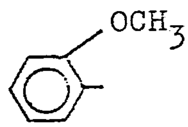 | 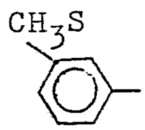 | H | $-CH_3$ | H | $n_D^{20}$ 1.6125 |
| 24 | 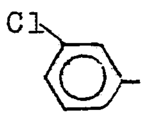 | 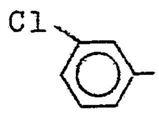 | H | $-CH_3$ | H | 155-158 |
| 25 | 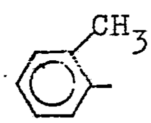 | 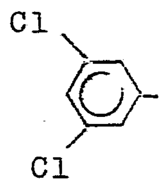 | H | $-CH_3$ | H | 141.5-143.5 |
| 26 | 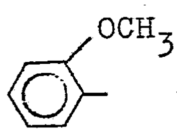 | 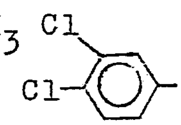 | H | $-CH_3$ | H | $n_D^{19}$ 1.5861 |
| 27 | " | 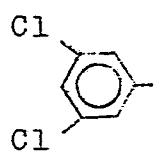 | H | $-CH_3$ | H | 156-157 |
| 28 | 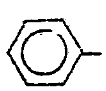 | 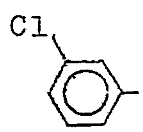 | $-CH_3$ | H | H | 93-95 |
| 29 | " | 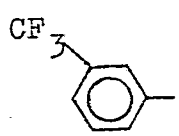 | $-CH_3$ | H | H | 98-100 |
| 30 | " | 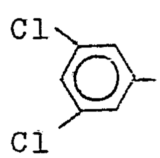 | $-CH_3$ | H | H | 106.5-109.5 |

Herstellung der Zwischen-Produkte der Formel (II)

Beispiel 31

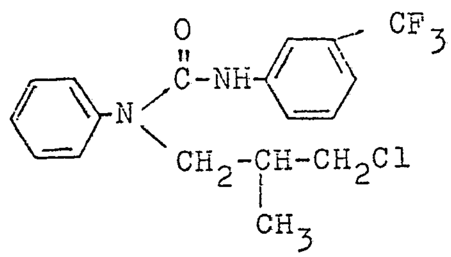

Bei Raumtemperatur wurden 18,7 g 3-Trifluoromethylphenylisocyanat zu einer Lösung von 18,4 g N-(3-Chloro-2-methylpropyl)anilin in 100 ml Chloroform hinzugefügt. Die Mischung wurde dann 1 h bei 40°C gerührt. Nach dem Entfernen des Chloroforms unter vermindertem Druck wurden 37 g N-/¯3-Chloro-2-methylpropyl_7-N-phenyl-N'-3-trifluoromethylphenylharnstoff erhalten. Der Schmelzpunkt des Produkts betrug 72-75°C.

Die N,N'-Diaryl-N-halogenoalkylharnstoffe der folgenden Beispiele 32 bis 36 wurden im wesentlichen wie in dem vorhergehenden Beispiel 31 beschrieben hergestellt.

| Beispiel Nr. | Verbindung | Schmp. (°C) /Brechungs-Index $n_D^{20}$ |
|---|---|---|
| 32 | N-(3-Chloro-2-methylpropyl)-N-phenyl-N'-3-chlorophenylharnstoff | 99-100 |
| 33 | N-(3-Chloro-2-methylpropyl)-N-2-methoxyphenyl-N'-3-trifluoromethylphenylharnstoff | 1,5343 |
| 34 | N-(3-Chloro-2-methylpropyl)-N-phenyl-N'-3-methylthiophenylharnstoff | 1,5974 |
| 35 | N-(3-Chloro-2-methylpropyl)-N-2-chlorophenyl-N'-3-chlorophenylharnstoff | 1,5681 |
| 36 | N-(3-Chloro-2-methylpropyl)-N-2-chlorophenyl-N'-3-trifluoromethylphenylharnstoff | 1,5331 |

Die in den folgenden Beispielen 37 bis 62 angegebenen N,N'-Diaryl-N'-halogenoalkylharnstoffe wurden in gleicher Weise, unter Verwendung der aufgeführten Ausgangsstoffe, im wesentlichen nach dem in dem vorhergehenden Beispiel 31 beschriebenen Verfahren hergestellt.

| Beispiel Nr. | Ausgangsstoff | Ausgangsstoff | Produkt |
|---|---|---|---|
| 37 | N-(3-Chloro-2-methyl-propyl)-3-fluoroanilin | Phenylisocyanat | N-(3-Chloro-2-methylpropyl)-N-3-fluorophenyl-N'-phenyl-harnstoff |
| 38 | N-(3-Chloro-2-methyl-propyl)-3-bromoanilin | Phenylisocyanat | N-(3-Chloro-2-methylpropyl)-N-3-bromophenyl-N'-phenyl-harnstoff |
| 39 | N-(3-Bromo-2-methyl-propyl)-3-nitroanilin | Phenylisocyanat | N-(3-Bromo-2-methylpropyl)-N-3-nitrophenyl-N'-phenyl-harnstoff |
| 40 | N-(3-Bromo-2-methyl-propyl)anilin | 3-Phenoxyphenyl-isocyanat | N-(3-Bromo-2-methylpropyl)-N-phenyl-N'-3-phenoxyphe-nylharnstoff |
| 41 | N-(3-Bromo-2-methyl-propyl)-3,4-dichloro-anilin | Phenylisocyanat | N-(3-Bromo-2-methylpropyl)-N-3,4-dichlorophenyl-N'-phenylharnstoff |
| 42 | N-(3-Chloro-2-methyl-propyl)anilin | 3,5-Dichlorophenyl-isocyanat | N-(3-Chloro-2-methylpropyl)-N-phenyl-N'-3,5-dichlorophe-nylharnstoff |

| Beispiel Nr. | Ausgangsstoff | Ausgangsstoff | Produkt |
|---|---|---|---|
| 43 | N-(3-Chloro-2-methylpropyl)anilin | 3,5-Xylylisocyanat | N-(3-Chloro-2-methylpropyl)-N-phenyl-N'-3,5-xylylharnstoff |
| 44 | N-(3-Bromo-2-methylpropyl)anilin | 3,5-Dimethoxyphenylisocyanat | N-(3-Bromo-2-methylpropyl)-N-phenyl-N'-3,5-dimethoxyphenylharnstoff |
| 45 | N-(3-Chloro-2-methylpropyl)-α-naphthylamin | Phenylisocyanat | N-(3-Chloro-2-methylpropyl)-N-α-naphthyl-N'-phenylharnstoff |
| 46 | N-(3-Bromo-2-methylpropyl)-2,4,5-trichloroanilin | Phenylisocyanat | N-(3-Bromo-2-methylpropyl)-N-2,4,5-trichlorophenyl-N'-phenylharnstoff |
| 47 | N-(3-Chloro-2-methylpropyl)anilin | 3-Methoxyphenylisocyanat | N-(3-Chloro-2-methylpropyl)-N-phenyl-N'-3-methoxyphenylharnstoff |
| 48 | N-(3-Bromo-2-methylpropyl)-2-toluidin | 3-Chlorophenylisocyanat | N-(3-Bromo-2-methylpropyl)-N-2-tolyl-N'-3-chlorophenylharnstoff |

| Beispiel Nr. | Ausgangsstoff | Ausgangsstoff | Produkt |
|---|---|---|---|
| 49 | N-(3-Chloro-2-methyl-propyl)-2-toluidin | 3-Methylthio-phenylisocyanat | N-(3-Chloro-2-methylpropyl)-N-2-tolyl-N'-3-methylthio-phenylharnstoff |
| 50 | N-(3-Chloro-2-methyl-propyl)-2-toluidin | 3-Trifluoromethyl-phenylisocyanat | N-(3-Chloro-2-methylpropyl)-N-2-tolyl-N'-3-trifluorome-thylphenylharnstoff |
| 51 | N-(3-Chloro-2-methyl-propyl)-2-methoxyanilin | 3-Chlorophenyl-isocyanat | N-(3-Chloro-2-methylpropyl)-N-2-methoxyphenyl-N'-3-chloro-phenylharnstoff |
| 52 | N-(3-Chloro-2-methyl-propyl)-2-methoxyanilin | 3-Tolylisocyanat | N-(3-Chloro-2-methylpropyl)-N-2-methoxyphenyl-N'-3-tolyl-harnstoff |
| 53 | N-(3-Bromo-2-methyl-propyl)-2-methoxyanilin | 3-Methylthio-phenylisocyanat | N-(3-Bromo-2-methylpropyl)-N-2-methoxyphenyl-N'-3-methyl-thiophenylharnstoff |
| 54 | N-(3-Chloro-2-methyl-propyl)-3-chloroanilin | 3-Chlorophenyl-isocyanat | N-(3-Chloro-2-methylpropyl)-N,N'-bis-(3-chlorophenyl)harn-stoff |

| Beispiel Nr. | Ausgangsstoff | Ausgangsstoff | Produkt |
|---|---|---|---|
| 55 | N-(3-Bromo-2-methyl-propyl)-2-toluidin | 3,5-Dichlorophenyl-isocyanat | N-(3-Bromo-2-methylpropyl)-N-2-tolyl-N'-3,5-dichloro-phenylharnstoff |
| 56 | N-(3-Bromo-2-methyl-propyl)-2-methoxyanilin | 3,4-Dichlorophenyl-isocyanat | N-(3-Bromo-2-methylpropyl)-N-2-methoxyphenyl-N'-3,4-di-chlorophenylharnstoff |
| 57 | N-(3-Chloro-2-methyl-propyl)-2-methoxyanilin | 3,5-Dichlorophenyl-isocyanat | N-(3-Chloro-2-methylpropyl)-N-2-methoxyphenyl-N'-3,5-di-chlorophenylharnstoff |
| 58 | N-(3-Chloro-1-methyl-propyl)anilin | 3-Chlorophenyl-isocyanat | N-(3-Chloro-1-methylpropyl)-N-phenyl-N'-3-chlorophenyl-harnstoff |
| 59 | N-(3-Chloro-2-methyl-propyl)anilin | 3-Trifluoromethyl-phenylisocyanat | N-(3-Chloro-2-methylpropyl)-N-phenyl-N'-3-trifluoromethyl-phenylharnstoff |
| 60 | N-(3-Chloro-1-methyl-propyl)anilin | 3,5-Dichlorophenyl-isocyanat | N-(3-Chloro-1-methylpropyl)-N-phenyl-N'-3,5-dichlorophe-nylharnstoff |

| Beispiel Nr. | Ausgangsstoff | Ausgangsstoff | Produkt |
|---|---|---|---|
| 61 | N-(3-Bromo-2-methylpropyl)anilin | 3-Trifluoromethylphenylisocyanat | N-(3-Bromo-2-methylpropyl)-N-phenyl-N'-3-trifluoromethylphenylharnstoff |
| 62 | N-(3-Bromo-2-methylpropyl)-2-methoxyanilin | 3-Trifluoromethylphenylisocyanat | N-(3-Bromo-2-methylpropyl)-N-2-methoxyphenyl-N'-3-trifluoromethylphenylharnstoff |

## P a t e n t a n s p r ü c h e

1. Methyl-substituierte Tetrahydro-2-pyrimidinon-Derivate der allgemeinen Formel

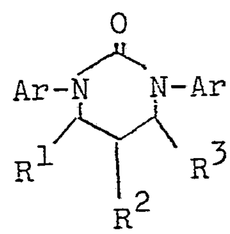

(I)

in der

Ar jeweils unabhängig voneinander für eine Aryl-Gruppe steht, die gegebenenfalls mono- oder polysubstituiert ist durch einen oder mehrere Substituenten ausgewählt aus Halogen, $C_1$- bis $C_6$-Alkyl, $C_1$- bis $C_6$-Alkoxy, $C_1$- bis $C_6$-Alkylthio, Nitro, Phenoxy und Trifluoromethyl, und

$R^1$, $R^2$ und $R^3$ unabhängig voneinander für ein Wasserstoff-Atom oder eine Methyl-Gruppe stehen, mit der Maßgabe, daß mindestens einer der Substituenten $R^1$, $R^2$ und $R^3$ eine Methyl-Gruppe darstellt.

2. Verbindungen nach Anspruch 1, dadurch gekennzeichnet, daß

Ar für eine Phenyl- oder -Naphthyl-Gruppe steht, die gegebenenfalls mono- oder polysubstituiert ist durch einen oder mehrere Substituenten ausgewählt aus Fluor, Chlor, Brom, Iod, Methyl, Ethyl, Propyl, Isopropyl, n-Butyl, Isobutyl, sec-Butyl, tert-Butyl,

Methoxy, Ethoxy, Propoxy, Isopropoxy, n-Butoxy, Isobutoxy, sec-Butoxy, tert-Butoxy, Methylthio, Ethylthio, Propylthio, Isopropylthio, n-Butylthio, Isobutylthio, sec-Butylthio, tert-Butylthio, Nitro, Phenoxy und Trifluoromethyl, und

$R^1$, $R^2$ und $R^3$ die in Anspruch 1 angegebenen Bedeutungen haben.

3. Verbindungen nach Anspruch 1 oder 2, dadurch gekennzeichnet, daß mindestens einer der Reste Ar einen Substituenten in der meta-Stellung trägt.

4. Verfahren zur Herstellung einer Verbindung nach Anspruch 1 bis 3, dadurch gekennzeichnet, daß eine Verbindung der allgemeinen Formel

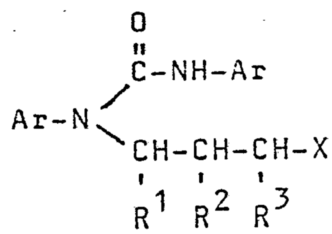

(II)

in der

Ar, $R^1$, $R^2$ und $R^3$ die in Anspruch 1 angegebenen Bedeutungen haben und

X für ein Halogen-Atom steht,

mit einem Alkalimetallhydroxid der allgemeinen Formel

MOH (III),

in der

M für ein Alkalimetall-Atom steht,

zur Umsetzung gebracht wird, gegebenenfalls in einem Lösungsmittel oder Verdünnungsmittel.

5. Herbizide Mittel, gekennzeichnet durch einen Gehalt an mindestens einem methyl-substituierten Tetrahydro-2-pyrimidinon-Derivat der Formel (I) gemäß Anspruch 1.

6. Verwendung von methyl-substituierten Tetrahydro-2-pyrimidinon-Derivaten der Formel (I) gemäß Anspruch 1 zur Bekämpfung von Unkräutern.

7. Verfahren zur Herstellung von herbiziden Mitteln, dadurch gekennzeichnet, daß man methyl-substituierte Tetrahydro-2-pyrimidinon-Derivate der Formel (I) gemäß Anspruch 1 mit Streckmitteln und/oder oberflächenaktiven Mitteln vermischt.

8. N,N'-Diaryl-N-halogenoalkylharnstoffe der allgemeinen Formel

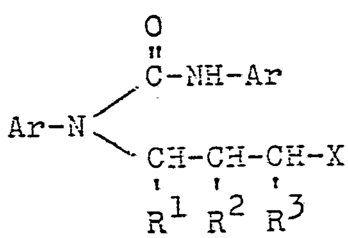

(II)

in der
X ein Halogen-Atom bezeichnet,
Ar jeweils unabhängig voneinander für eine Aryl-Gruppe steht, die gegebenenfalls mono- oder polysubstituiert ist durch einen oder mehrere Substituenten ausgewählt aus Halogen, $C_1$- bis $C_6$-Alkyl, $C_1$- bis $C_6$-Alkoxy, $C_1$- bis $C_6$-Alkylthio, Nitro, Phenoxy und Trifluoromethyl, und
$R^1$, $R^2$ und $R^3$ unabhängig voneinander für ein Wasserstoff-Atom oder eine Methyl-Gruppe stehen, mit der Maßgabe, daß mindestens einer der Substituenten $R^1$, $R^2$ und $R^3$ eine Methyl-Gruppe darstellt.

9. Verfahren zur Herstellung einer Verbindung nach Anspruch 8, dadurch gekennzeichnet, daß ein Amin der allgemeinen Formel

$$\text{Ar-NH-}\underset{\displaystyle |}{\overset{R^1}{\text{CH}}}\text{-}\overset{R^2}{\text{CH}}\text{-}\overset{R^3}{\text{CH}}\text{-X} \qquad (IV),$$

in der Ar, $R^1$, $R^2$ und $R^3$ die in Anspruch 8 angegebenen Bedeutungen haben und X für ein Halogen-Atom steht, mit einem Isocyanat der allgemeinen Formel

$$\text{Ar-N=C=O} \qquad (V),$$

in der Ar die in Anspruch 8 angegebene Bedeutung hat und gleich dem oder verschieden von dem Ar-Rest in dem Amin der Formel (IV) sein kann, zur Umsetzung gebracht wird.